# DEMANDE DE BREVET EUROPEEN

(11) **EP 1 092 432 A1**
(43) Date de publication de la demande: **18.04.2001**
(21) Numéro de dépôt: 99870212.0
(22) Date de dépôt: 15.10.1999
(51) Int. Cl.: A61K 31/165, A61K 31/225, A61K 31/365, C07D 307/93, C07C 69/38, C07C 233/07, C07C 235/74, A61P 9/10

(54) **Composes anti-ischemiques**

(71) Demandeur: SOCIETE DE CONSEILS DE RECHERCHES ET D'APPLICATIONS SCIENTIFIQUES S.A. (S.C.R.A.S.), 75016 Paris (FR)
(72) Inventeur: Michiels, Carine, 5190 SPY (BE); Redon, Martine, 5100 Jambes (BE); Remacle, José, 5020 Malonne (BE)
(74) Mandataire: Van Malderen, Joelle

(57) **Abrégé**

La présente invention concerne un composé de formule (I), un sel éventuel ou une prodrogue de celui-ci correspondant à la formule I : 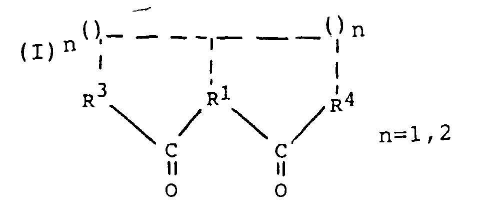 ainsi que son application pour la prévention et/ou le traitement de l'ischémie partielle ou totale ou de pathologies associées à l'ischémie ou associées à des déficiences mitochondriales.

## Description

### Objet de l'invention

La présente invention est relative à des nouveaux agents thérapeutiques ayant un effet protecteur sur les complexes protéiniques de la membrane mitochondriale interne, et qui peuvent être utilisés de préférence pour la prévention et/ou le traitement de l'ischémie partielle ou totale, de pathologies associées à l'ischémie ou associées à des déficiences mitochondriales, ou de l'apoptose.

### Arrière-plan technologique à la base de l'invention

L'ischémie est une interruption de l'irrigation sanguine d'un organe ou d'un tissu par oblitération, compression ou spasme d'une artère. Les conséquences dépendent de la nature du tissu privé de sang oxygéné et de la durée du phénomène. L'ischémie totale provoque la mort du tissu en un temps variable selon ses besoins en oxygène. L'ischémie partielle provoque soit une diminution des capacités de l'organe (ralentissement psychique, insuffisance des fonctions hépatique ou rénale, etc.), soit l'incapacité de modifier son travail à toute stimulation, faisant apparaître alors des signes de souffrance (crises d'angine de poitrine, crampes de l'artéritique). L'ischémie est très nocive pour le tissu nerveux. Elle se traduit dans un premier temps par des perturbations métaboliques et fonctionnelles réversibles qui peuvent donner lieu à une activité nerveuse anormale par exemple une crise d'épilepsie ou certaines lésions ischémique du cortex cérébral. Après quelques minutes d'ischémie, des lésions irréversibles du tissu nerveux s'installent, avec gonflement puis nécrose des corps cellulaires. C'est l'infarctus cérébral ou ramollissement cérébral.

Il est connu d'utiliser des molécules agonistes des récepteurs alpha-adrénergiques et agonistes des récepteurs alpha-pré-synaptiques pour le traitement de l'ischémie. Ces composés sont utilisés au niveau cardiaque pour le traitement de l'angor, qui est une expression clinique de l'ischémie myocardique aiguë, et qui est le résultat d'un déséquilibre momentané entre la demande en oxygène du myocarde et l'apport en oxygène par la circulation, pouvant conduire dans les cas graves à un infarctus du myocarde.

La demande internationale de brevet W098/51291 décrit l'utilisation de différents extraits de plantes pour le traitement et/ou la prévention de l'ischémie ou de pathologies associées à l'ischémie ou à un déficit énergétique. Ce document décrit également le mécanisme d'action de ces molécules qui permettent une inhibition de la cascade d'activation des cellules endothéliales induite par l'hypoxie ainsi que l'effet protecteur de ces produits sur certaines déficiences mitochondriales, en particulier par un effet protecteur des complexes I et III des mitochondries. Ce document propose un modèle biochimique permettant d'obtenir *in vitro* un effet protecteur de composés anti-ischémiques.

### Buts de l'invention

La présente invention vise à fournir de nouveaux composés destinés à induire un effet protecteur sur les complexes protéiniques de la membrane mitochondriale interne et à être utilisés en particulier dans la prévention ou le traitement de l'ischémie partielle ou totale, de pathologies liées à l'ischémie ou de pathologies liées à des déficiences mitochondriales.

Un but particulier de la présente invention est de fournir des composés ayant une activité améliorée et/ou ne présentant pas les effets secondaires des molécules de l'état de la technique.

### Eléments caractéristiques de l'invention

La présente invention concerne en particulier des nouveaux composés de formule I, leurs sels éventuels ou des prodrogues de tels composés : 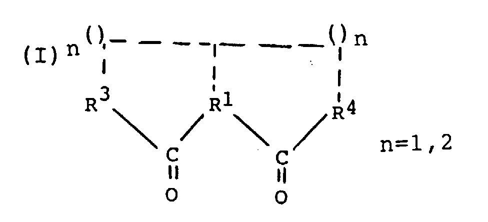 dans laquelle :
- R¹ est CH₂, NH, ou un ligand, de préférence un atome de carbone, formant avec R³ et/ou R⁴. un cycle aromatique ou non aromatique de 5 ou 6 atomes de carbone, comportant éventuellement sur chaque cycle un hétéroatome, de préférence un atome d'azote, d'oxygène ou de soufre;
- R³ et/ou R⁴ représentent :
   - une amine de formule 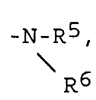 ou
   - un groupement de formule -Z-R⁶ dans lesquels :
      - Z représente O ou S,
      - R⁵ représente H ou un alkyle de 1 à 6 atomes de carbone (de préférence, R⁵ représente un méthyle ou un éthyle),
      - R⁶ représente un groupement hydrophobe de préférence terbutyle, allyle ou un cycle aromatique ou non aromatique de 5, 6 ou 7 atomes de carbone comprenant éventuellement un ou plusieurs hétéroatomes et dans lequel les atomes de carbone sont éventuellement substitués par :
         - un groupe alkyle R⁷ de 1 à 6 atomes de carbone (de préférence, un méthyle ou un éthyle),
         - un groupement cétone,
         - un groupement hydroxyle,
         - un groupement ester,
         - un élément halogène (F, Cl, Br ou I),
         - un trifluorométhyle (CF₃), ou
         - un groupement de formule 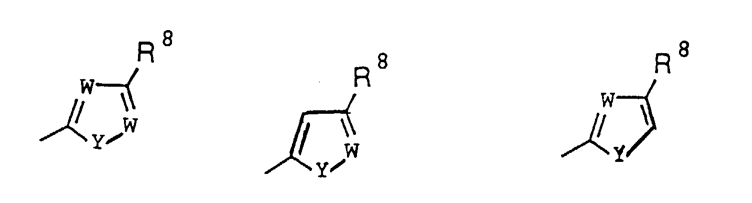
         - dans lequel :
            - W représente CH ou un atome d'azote,
            - Y représente S ou O, et
            - R⁸ représente un groupe alkyle de 1 à 6 atomes de carbone, de préférence un méthyle ou un éthyle;
            - avec la condition supplémentaire que si R³ forme avec R¹ ledit cycle susmentionné ou que si R³ est soit une amine de formule 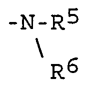 ou le groupement de formule -Z-R⁶ susmentionné, R⁴ représente un alkyle de 1 à 10 atomes de carbone, saturé ou insaturé, comprenant éventuellement un ou plusieurs hétéroatomes. Dans ledit alkyle, un ou plusieurs carbones peuvent éventuellement être substitués par un élément halogène (fluor, chrome, brome ou iode) ou un trifluorométhyle. De préférence, l'alkyle représente un propène.

Dans les composés de formule I, R³ et R⁴ représentent de préférence une amine de formule 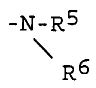 ou un groupement de formule -Z-R⁶ dans lequel R⁵ représente H ou un méthyle, Z représente O ou S (de préférence O), et R⁶ représente un cyclopentène, un cyclohexane ou un groupement benzène.

Ces cycles aromatiques ou non aromatiques comportent éventuellement un ou plusieurs hétéroatomes, de préférence un ou plusieurs atomes d'oxygène ou de soufre, et dans lesquels les atomes de carbone sont substitués par des groupes cétone, hydroxyle ou ester ou par un élément halogène tel que décrit ci-dessus.

Les composés préférés de l'invention sont choisis parmi le groupe constitué par le malonate de dicyclopenten-2-yle, le 2-propényl 3-oxa-2-oxobicyclo [3.1.0]hexane-1-carboxylate, le malonate de cyclopentèn-2-yle et d'éthyle, le malonate de cyclopentèn-2-yle et de méthyle, la N,N'-diphényl malonamide, le N-phénylamido éthanoate d'éthyle, le 2,6-diméthyl-N-phénylamido éthanoate d'éthyle et le N-méthyl-N-cyclohexylamido éthanoate d'éthyle, éventuellement leurs sels ou des prodrogues de ceux-ci.

La présente invention concerne également les sels d'addition acides non toxiques pharmaceutiquement acceptables des composés de l'invention comportant une amine. Comme exemples d'acides pharmaceutiquement acceptables, on peut citer les acides minéraux comme les acides chlorhydrique, bromhydrique, sulfurique, nitrique, phosphorique, etc. et les acides organiques comme les acides acétique, citrique, tartrique, benzoïque, salicylique, maléique, etc.

La présente invention concerne également la première application des composés de l'invention en tant que médicament, ainsi que les composés de l'invention, éventuellement leurs sels et les prodrogues desdits composés.

On entend par "prodrogues", des dérivés fonctionnels des composés de formule I qui peuvent être convertis, de préférence *in vivo,* chez le patient selon la forme requise de formule I. De telles prodrogues de ces composés peuvent être obtenues par les procédés bien connus de l'homme de l'art tels que ceux décrits dans le document "*Design* and *Prodrugs*", Ed. H. Bundgaard, Elsevier (1985). Les composés de l'invention concernent les deux formes énantiomériques, les différents isomères séparés ou un mélange d'entre eux.

Les nouveaux composés de l'invention sont avantageusement utilisés pour obtenir un effet protecteur sur les complexes protéiniques de la membrane mitochondriale interne des cellules d'un mammifère, de préférence d'un humain. Les composés de la présente invention trouvent en particulier une application dans la prévention et/ou le traitement de l'ischémie partielle ou totale, de pathologies associées à l'ischémie ou de pathologies associées à des déficiences mitochondriales.

On entend par "ischémie (partielle ou totale), pathologies associées à l'ischémie et pathologies associées à des déficiences mitochondriales", les maladies, de préférence vasculaires, choisies parmi le groupe constitué par l'infarctus du myocarde, l'ischémie cérébrale, l'insuffisance veineuse chronique, les artériopathies, c'est-à-dire des lésions dues à l'athérosclérose affectant les artères des patients, en particulier celles des membres inférieurs, le phénomène de Raynaud lié à des vasospasmes conduisant à une vasoconstriction des artères, les ulcères, la gangrène, l'altération de la perméabilité capillaire, la fragilité des capillaires, les cicatrisations, les altérations de la peau, les défauts rétiniens d'origine ischémique, la baisse d'acuité auditive d'origine ischémique, les troubles associés aux séjours en haute altitude, l'angine de poitrine engendrée par de courts moments d'obstruction coronarienne, l'hypertension pulmonaire, l'ischémie hépatique, la maladie de Parkinson, les myopathies et les syndromes associés à des problèmes vasculaires tels que le diabète, où une hypertension et une altération du flux sanguin apparaissent dans les membres inférieurs. Ces maladies et pathologies liées à l'ischémie sont bien connues des cliniciens et médecins, qui peuvent adapter l'utilisation de la composition pharmaceutique pour le traitement et/ou la prévention des symptômes et dysfonctionnements du corps humain ou animal associés aux maladies susmentionnées et/ou pour prévenir ou diminuer la possibilité d'en être atteint.

Les Inventeurs ont découvert de manière inattendue que ces différents syndromes ou maladies peuvent être traités par les composés de l'invention, leurs sels éventuels et des prodrogues de ceux-ci, et que ces composés agissent selon le même mode d'action biochimique.

Un autre aspect de la présente invention concerne une composition pharmaceutique comprenant un véhicule ou excipient pharmaceutique adéquat et une quantité suffisante d'un ou plusieurs des composés de l'invention, c'est-à-dire une quantité suffisante pour au moins améliorer ou prévenir les symptômes susmentionnés chez un mammifère, en particulier un humain. Cette quantité suffisante pourra varier en fonction de certains facteurs tels que l'état de condition de l'animal à traiter, du mode d'administration, de la sévérité des effets secondaires, la stabilité du composé dans le sérum ou le sang circulant, etc. De préférence, la quantité suffisante de composé utilisée dans la composition pharmaceutique de l'invention est comprise entre 0,1 et 200 mg/patient, de préférence entre 1 et 50 mg/patient, de préférence entre 20 et 30 mg/patient, cette quantité pouvant être adaptée en particulier en fonction des doses d'administration nécessaires et du poids du patient, comme cela peut être extrapolé à partir des exemples d'application *in vivo* ci-dessous.

La composition pharmaceutique comprend un véhicule pharmaceutique adéquat qui peut varier selon le mode d'administration et peut être éventuellement combiné avec un adjuvant de manière à améliorer les propriétés thérapeutiques du composé de l'invention ou pour réduire ses possibles effets secondaires. De tels véhicules pharmaceutiques adéquats ou adjuvants sont bien connus de l'homme de l'art et peuvent être préparés selon les procédures appliquées généralement par les pharmaciens et peuvent inclure tout véhicule pharmaceutique non toxique, solide, liquide ou gazeux. Le pourcentage de composé pharmaceutique peut varier en fonction de la fréquence d'administration et les éventuels effets secondaires sur l'animal, y compris l'humain.

Pour préparer de telles compositions pharmaceutiques sous forme de tablettes, il est possible d'incorporer des éléments tels que de l'amidon de maïs, du lactose, du sucrose, du sorbitol, du talc, de l'acide stéarique, du stéarate de magnésium, des gommes ou des diluants comprenant un pourcentage variable d'eau ou d'un solvant tel qu'un sirop, des suspensions huileuses ou aqueuses, des émulsions parfumées, etc. Des agents dispersables utilisés dans des compositions aqueuses peuvent comprendre des gommes, des alginates, des dextranes, des dérivés de carboxyméthyl cellulose, etc.

La présente invention concerne également un procédé de traitement thérapeutique ou préventif de l'ischémie ou des pathologies liées à l'ischémie telles que susmentionnées comprenant l'administration d'une quantité suffisante du composé de l'invention ou de la composition pharmaceutique de l'invention audit patient, de préférence un humain, de manière à prévenir, réduire ou supprimer les symptômes de l'ischémie, des pathologies liées à l'ischémie ou associées à des déficiences mitochondriales, ou l'apoptose.

Un dernier aspect de la présente invention concerne l'utilisation des composés de l'invention ou de la composition pharmaceutique de l'invention pour la préparation d'un médicament destiné au traitement ou à la prévention de l'ischémie, des pathologies liées à l'ischémie ou associées à des déficiences mitochondriales telles que mentionnées ci-dessus ou de l'apoptose.

La présente invention concerne également le procédé de préparation des composés de l'invention, comprenant les étapes suivantes :
- estérification d'un composé intermédiaire R³-H par un groupement de formule 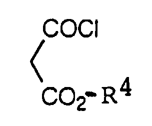 en présence de dicyclohexyl carbodiimide (DCC),
- éventuellement suivie d'une ou plusieurs réactions de cyclisation en présence d'un agent oxydant tel que de l'acétate de manganèse Mn (III) ou de l'acétate de cuivre Cu (II) monohydrate et éventuellement d'acétate de sodium.

Les définitions des radicaux mentionnés sont celles données dans la formule I pour les composés de l'invention.

Les différents aspects de la présente invention sont décrits en détails dans les exemples non limitatifs présentés ci-dessous.

### Exemples

### Exemple 1 : Synthèse de malonate de dicyclopentèn-2-yle

### Synthèse de 2-cyclopenténol

Cette synthèse est réalisée selon le procédé décrit par A.L. Gemal et J.L. Luche (J. Am. Chem. Soc. 103, p. 5454 (1981)).

A une solution de 2-cyclopenténone (5.00 g; 60.90 mmoles) dans le méthanol (commercial, 99%) est ajouté du chlorure de cérium heptahydrate (CeCl₃.7H₂O) (22.69 g; 60.90 mmoles) et le mélange réactionnel est agité à température ambiante pendant 40 minutes pour homogénéisation. Le mélange est alors refroidi à 0 °C et du borohydrure de sodium (NaBH₄) (2.30 g; 60.90 mmoles) est lentement additionné. Après addition complète, le mélange est agité à 0 °C pendant 3 heures. Une solution diluée d'acide chlorhydrique est alors ajoutée pour ajuster le pH à 7. Les phases sont séparées et la phase aqueuse est extraite plusieurs fois à l'éther (5x50 ml). Les phases organiques réunies sont séchées sur MgSO₄ anhydre et concentrées pour donner le 2-cyclopenténol (3.81 g, 74%).

### Synthèse du malonate de dicyclopentèn-2-yle

A une solution de 2-cyclopenténol (1.00 g; 11.89 mmoles) dans 50 ml de dichlorométhane sont additionnés successivement l'acide malonique (1.12 g; 10.81 mmoles) et la 4-diméthylamino pyridine (DMAP) (0.13 g; 1.08 mmoles). Le mélange réactionnel est agité sous atmosphère inerte et refroidi à 0 °C. A cette température, la dicyclohexyl carbodiimide (DCC) (2.34 g; 11.35 mmoles) est ajoutée en une seule portion. Un mélange blanc laiteux est obtenu qui est agité à 0 °C pendant 1 heure puis à température ambiante jusqu'à réaction complète (contrôle de l'évolution de la réaction par TLC, éluant Pentane/Acétate d'éthyle : 8/2). Le précipité est éliminé par filtration sur célite et lavé au dichlorométhane. Le filtrat est lavé avec une solution aqueuse saturée en NaHCO₃ (10 ml). Les phases sont séparées et la phase aqueuse est extraite au dichlorométhane (3x10 ml). Les phases organiques réunies sont lavées successivement avec une solution aqueuse saturée en NaCl et à l'eau, puis séchées sur MgSO₄ anhydre et concentrées. Le précipité est à nouveau éliminé par filtration sur célite et lavé au pentane. La purification du brut réactionnel est effectuée par chromatographie sur colonne de silice (élution Pentane/Acétate d'éthyle : 8/2) pour donner MRC2P119 (2.40 g; 94%) sous forme d'une huile incolore.

### Caractérisation du produit

¹H RMN (CDCl₃, 400 MHz) δ : 1.81-2.55 (m, 8H), 3.32 (s, 2H), 5.76-5.84 (m, 4H), 6.12 (m, 2H); ¹³C RMN (CDCl₃, 175 MHz) δ : 29.05, 30.53, 41.36, 80.97, 128.33, 137.44, 165.92; IR (film) : 3450, 3062, 2947, 2858, 1727, 1618, 1454, 1411, 1348, 1319, 1270, 1151, 1029, 973, 917, 878, 783, 735 cm⁻¹; PM : 236.27; MS m/z 171 (M⁺· - 65); Analyse Elémentaire calculé C (66.09%), H (6.82%); trouvé C (66.09%), H (6.90%).

### Exemple 2 : Synthèse du 2-propényl 3-oxa-2-oxobicyclo [3.1.0]hexane-1-carboxylate

### Synthèse du diallyl malonate

A une solution d'acide malonique (5.00 g; 48.05 mmoles) dans 330 ml de dichlorométhane sont additionnés successivement l'alcool allylique (10.51 g; 124.93 mmoles) et la DMAP (1.17 g; 9.61 mmoles). Le mélange réactionnel est agité sous atmosphère inerte et refroidi à 0 °C. A cette température, la DCC (20.82 g; 100.90 mmoles) est ajoutée en une seule portion. Un mélange blanc laiteux est obtenu qui est agité à 0 °C pendant 1 heure puis à température ambiante jusqu'à réaction complète (contrôle de l'évolution de la réaction par TLC; éluant Pentane/Acétate d'éthyle : 9/1). Le précipité est éliminé par filtration sur célite et lavé au dichlorométhane. Le filtrat est lavé avec une solution aqueuse saturée en NaHCO₃ (20 ml). Les phases sont séparées et la phase aqueuse est extraite au dichlorométhane (3x20 ml). Les phases organiques réunies sont lavées successivement avec une solution aqueuse saturée en NaCl et à l'eau, puis séchées sur MgSO4 anhydre et concentrées. Le précipité est à nouveau filtré sur célite et lavé au pentane. La purification est effectuée par chromatographie sur colonne de silice (élution Pentane/Acétate d'éthyle : 9/1) pour donner le composé de l'invention (MRC2PSS) (8.09 g; 92%) sous forme d'une huile incolore.

### Caractérisation du produit

¹H RMN (CDCl₃, 400 MHz) δ : 3.44 (s, 2H), 4.64 (d, J=5.9 Hz, 4H), 5.24 (d, J=10.3 Hz, 2H), 5.34 (d, J=17.1 Hz, 2H), 5.91 (ddt, J=17.1-10.3-5.9 Hz, 2H); ¹³C RMN (CDCl₃, 175 MHz) δ : 40.99, 65.57, 118.20, 131.26, 165.68; IR (film) : 3653, 3089, 2989, 2951, 1739, 1650, 1450, 1414, 1368, 1330, 1274, 1150, 995, 935, 631 cm⁻¹; PM : 184.19; MS m/z 184 (M⁺·); Analyse Elémentaire calculé C (58.69%), H (6.57%); trouvé C (58.66%), H (6.57%).

### Synthèse du 2-propényl 3-oxa-2-oxobicyclo [3.1.0]hexane-1-carboxylate

A une solution de malonate de diallyle (MRC2P55) (2.00 g; 10.86 mmoles) dans 55 ml d'acide acétique glacial sont additionnés successivement l'acétate de manganèse (III) dihydrate (Mn(OAC)₃.2H₂O) (11.64 g; 43.43 mmoles), l'acétate de Cuivre (II) monohydrate (Cu(OAc)₂.H₂O) (4.34 g; 21.72 mmoles) et l'acétate de sodium (AcONa) (1.78 g; 21.72 mmoles). La solution brune obtenue est chauffée à 80 °C pendant 20 heures (ceci correspond à la disparition de la coloration brune et à l'apparition d'une coloration bleue turquoise). L'évolution de la réaction est contrôlée par TLC (éluant Pentane/Acétate d'éthyle : 9/1). Le précipité est éliminé par filtration sur célite et lavé au dichlorométhane. Le filtrat est lavé à l'eau (30 ml). La phase aqueuse est extraite au dichlorométhane (2x20 ml). Les phases organiques réunies sont lavées successivement à l'eau (2x20 ml) et avec une solution aqueuse saturée en NaHCO₃ (2x20 ml) puis séchées sur MgSO₄ anhydre et concentrées. Le brut réactionnel est purifié par chromatographie sur colonne de silice (élution Pentane/Acétate d'éthyle : 1/1) pour donner le composé de l'invention (MCR2PS7) (0.49 g; 25%) sous forme d'une huile incolore.

### Caractérisation du produit

¹H RMN (CDCl₃, 400 MHz) δ : 1.41 (dd, J=5.4-4.9 Hz, 1H), 2.10 (dd, J=8.0-4.6 Hz, 1H), 2.79 (m, 1H), 4.20 (d, J=9.3 Hz, 1H), 4.38 (dd, J=9.3-4.9 Hz, 1H), 4.68 (d, J=5.4 Hz, 2H), 5.28 (dd, J=10.2-1.5 Hz, 1H), 5.39 (dd, J=17.1-1.5 Hz, 1H), 5.94 (ddt, J=17.1-10.2-5.4 Hz, 1H); ¹³C RMN (CDCl₃, 175 MHz) δ : 20.56, 27.82, 29.09, 65.97, 66.85, 118.60, 131.10, 166.14, 170.20; IR (film) : 3653, 3094, 2989, 1777, 1725, 1650, 1447, 1393, 1314, 1271, 1183, 1115, 1090, 1047, 998, 939, 847, 793, 765, 703, 632 cm⁻¹; PM : 182.18; MS m/z 183 (MH⁺·); Analyse Elémentaire calculé C (59.34%), H (5.53%); trouvé C (59.28%), H (5.68%).

### Exemple 3 : Synthèse du malonate de cyclopentèn-2-yle et d'éthyle

### Synthèse de l'acide éthyl malonique

### Etape 1

Dans un ballon tricol de 250 ml, équipé d'une ampoule à addition et d'un réfrigérant, on solubilise le malonate de diéthyle (20.00 g; 124.87 mmoles) dans 80 ml d'éthanol (commercial). Le mélange incolore obtenu est agité à température ambiante et une solution de KOH (7.00 g; 124.76 mmoles) dans l'éthanol (80 ml) est lentement ajoutée (durée de l'addition : 1 h). Après addition complète, le mélange obtenu est agité à température ambiante pendant une nuit (précipitation du malonate de potassium et d'éthyle). La précipitation du sel est avantageusement accélérée en refroidissant le ballon réactionnel au bain de glace. Le sel de potassium est récupéré par filtration, lavé avec de faibles quantités d'éther puis séché sous pression réduite (17.77 g; 84%).

### Etape 2

Dans un ballon tricol de 250 ml, équipé d'un agitateur magnétique, d'une ampoule à addition et d'un thermomètre, on solubilise le malonate de potassium et d'éthyle (17.77 g; 104.49 mmoles) dans 18 ml d'eau. Le mélange réactionnel est refroidi par l'intermédiaire d'un bain de glace et 8.2 ml d'acide chlorhydrique concentré sont lentement ajoutés. Le mélange réactionnel est filtré et le précipité de KCl est lavé à l'éther (3x50 ml). Le filtrat est décanté et la phase aqueuse est extraite à l'éther (3x50 ml). Les phases éthérées réunies sont séchées sur MgSO₄ anhydre, filtrées et concentrées pour donner l'acide éthyl malonique (13.45 g; 82% rendement global pour les deux étapes) sous forme d'une huile incolore.

### Synthèse du malonate de cyclopentèn-2-yle et d'éthyle

A une solution de 2-cyclopenténol (0.70 g; 8.32 mmoles) dans 30 ml de dichlorométhane sont additionnés successivement l'acide éthyl malonique (0.85 g; 6.40 mmoles) et la DMAP (0.08 g; 0.64 mmoles). Le mélange réactionnel est agité sous atmosphère inerte et refroidi à 0 °C. A cette température, la DCC (1.39 g; 6.72 mmoles) est ajoutée en une seule portion. Un mélange blanc laiteux est obtenu qui est agité à 0 °C pendant 1 heure puis à température ambiante jusqu'à réaction complète (contrôle de l'évolution de la réaction par TLC, éluant Pentane/Acétate d'éthyle : 8/2). Le précipité est éliminé par filtration sur célite et lavé au dichlorométhane. Le filtrat est lavé avec une solution aqueuse saturée en NaHCO₃ (10 ml). Les phases sont séparées et la phase aqueuse est extraite au dichlorométhane (3x10 ml). Les phases organiques réunies sont lavées successivement avec une solution aqueuse saturée en NaCl et à l'eau, puis séchées sur MgSO₄ anhydre et concentrées. Le précipité est à nouveau éliminé par filtration sur célite et lavé au pentane. La purification du brut réactionnel est effectuée par chromatographie sur colonne de silice (élution Pentane/Acétate d'éthyle : 8/2) pour donner le composé de l'invention (MCR2P45) (1.39 g; 84%) sous forme d'une huile incolore.

### Caractérisation du produit

¹H RMN (CDCl₃, 400 MHz) δ : 1.28 (t, J=7.3 Hz, 3H), 1.83 (m, 1H), 2.31 (m, 2H), 2.50 (m, 1H), 3.34 (s, 1H), 4.20 (q, J=7.3 Hz, 2H), 5.76 (m, 1H), 5.83 (m, 1H), 6.13 (m, 1H); ¹³C RMN (CDCl₃, 175 MHz) δ : 13.41, 29.00, 30.47, 41.10, 60.64, 81.07, 128.46, 165.87, 165.94; IR (film) : 3451, 2983, 1750, 1731, 1459, 1412, 1370, 1346, 1323, 1271, 1189, 1151, 1032, 971, 917, 879, 736 cm⁻¹; PM : 198.22; MS m/z 170 (M⁺· - 28) .

### Exemple 4 : Synthèse du malonate de cyclopentèn-2-yle et de méthyle

### Synthèse de l'acide méthyl malonique

### Etape 1

Dans un ballon tricol de 100 ml, équipé d'une ampoule à addition et d'un réfrigérant, on solubilise du malonate de diméthyle (10.00 g; 75.69 mmoles) dans 40 ml de méthanol (commercial). Le mélange incolore obtenu est agité à température ambiante et une solution de KOH (4.25 g; 75.69 mmoles) dans le méthanol (40 ml) est lentement ajoutée (durée de l'addition : 1 h). Après addition complète, le mélange obtenu est agité à température ambiante pendant une nuit (précipitation du malonate de potassium et de méthyle). La précipitation du sel peut être accélérée en refroidissant le ballon réactionnel au bain de glace. Le sel de potassium est récupéré par filtration, lavé avec de faibles quantités d'éther puis séché sous pression réduite (10.27 g; 87%).

### Etape 2

Dans un ballon tricol de 50 ml, équipé d'un agitateur magnétique, d'une ampoule à addition et d'un thermomètre, on solubilise le malonate de potassium et de méthyle (10.27 g; 65.76 mmoles) dans 10 ml d'eau. Le mélange réactionnel est refroidi par l'intermédiaire d'un bain de glace et 5.5 ml d'acide chlorhydrique concentré sont lentement ajoutés. Le mélange réactionnel est filtré et le précipité de KCl est lavé à l'éther (3x10 ml). Le filtrat est décanté et la phase aqueuse est extraite à l'éther (3x10 ml). Les phases éthérées réunies sont séchées sur MgSO₄ anhydre, filtrées et concentrées pour donner l'acide méthyl malonique (7.42 g; 96%) sous forme d'une huile incolore.

### Synthèse du malonate de cyclopentèn-2-yle et de méthyle

A une solution de 2-cyclopenténol (1.00 g; 11.89 mmoles) dans 35 ml de dichlorométhane sont additionnés successivement l'acide méthyl malonique (1.08 g; 9.15 mmoles) et la DMAP (0.11 g; 0.91 mmoles). Le mélange réactionnel est agité sous atmosphère inerte et refroidi à 0 °C. A cette température, la DCC (1.98 g; 9.60 mmoles) est ajoutée en une seule portion. Un mélange blanc laiteux est obtenu qui est agité à 0 °C pendant 1 heure puis à température ambiante jusqu'à réaction complète (contrôle de l'évolution de la réaction par TLC, éluant Pentane/Acétate d'éthyle : 8/2). Le précipité est éliminé par filtration sur célite et lavé au dichlorométhane. Le filtrat est lavé avec une solution aqueuse saturée en NaHCO₃ (10 ml). Les phases sont séparées et la phase aqueuse est extraite au dichlorométhane (3x10 ml). Les phases organiques réunies sont lavées successivement avec une solution aqueuse saturée en NaCl et à l'eau, puis séchées sur MgSO₄ anhydre et concentrées. Le précipité est à nouveau éliminé par filtration sur célite et lavé au pentane. La purification du brut réactionnel est effectuée par chromatographie sur colonne de silice (élution Pentane/Acétate d'éthyle : 8/2) pour donner le composé de l'invention (MCR2P85) (1.40 g; 83%) sous forme d'une huile incolore.

### Caractérisation du produit

¹H RMN (CDCl₃, 400 MHz) δ : 1.85 (m, 1H), 2.29 (m, 2H), 2.51 (m, 1H), 3.36 (s, 2H), 3.75 (s, 3H), 5.76 (m, 1H), 5.83 (m, 1H), 6.13 (m, 1H); ¹³C RMN (CDCl₃, 175 MHz) δ : 29.27, 30.73, 41.16, 52.04, 81.43, 128.44, 137.83, 166.10, 166.74; IR (film) : 2955, 2858, 1736, 1438, 1412, 1348, 1273, 1152, 1028, 968, 917, 878, 737 cm⁻¹; PM : 184.19; MS m/z 156 (M⁺· - 28); Analyse Elémentaire calculé C (58.69%), H (6.57%); trouvé C (58.60%), H (6.59%).

### Exemple 5 : Synthèse de la N,N'-diphényl malonamide

A une solution de N-phényl amine (0.75 ml; 8.22 mmoles) dans 25 ml de dichlorométhane est additionnée la triéthylamine (0.72 ml; 5.14 mmoles). Le mélange incolore obtenu est refroidi à 0 °C par l'intermédiaire d'un bain de glace et on y ajoute lentement à la seringue le dichlorure de malonyle (0.5 ml; 5.14 mmoles). Une solution rouge/orange intense accompagnée d'un précipité est obtenue. Le mélange réactionnel est agité à 0 °C pendant 2 heures (contrôle de l'évolution de la réaction par TLC) (éluant Pentane/Acétate d'éthyle : 1/9), puis, environ 4 ml d'une solution de HCl 10% sont additionnés. Les phases sont séparées et la phase organique est lavée successivement à l'eau (10 ml) et avec une solution aqueuse saturée en NaCl (10 ml). Après séchage sur MgSO₄ anhydre, filtration et concentration, on isole un solide orangé. La purification est effectuée par recristallisation dans un mélange Pentane/Acétate d'éthyle pour fournir le composé de l'invention (MCR2P219) (0.33 g; 32%) sous forme d'une poudre couleur écru.

### Caractérisation du produit

¹H RMN (DMSO-d₆, 400 MHz) δ : 3.47 (s, 2H), 7.06 (m, 2xlH), 7.31 (m, 2x2H), 7.60 (m, 2x2H), 10.17 (s, 2H); ¹³C RMN (DMSO-d₆, 175 MHz) δ : 37.20, 110.30, 114.60, 120.00, 130.20, 156.60; IR (pastille KBr) : 3274, 3152, 3066, 1669, 1650, 1599, 1560, 1538, 1500, 1444, 1416, 1358, 1309, 1294, 1251, 1193, 1162, 979, 905, 857, 752, 693, 618 cm⁻¹; m.p. = 220 °C; PM : 254.29.

### Exemple 6 : Synthèse du N-phénylamido éthanoate d'éthyle

### Synthèse du chloroformyl éthanoate d'éthyle

Dans un ballon tricol de 50 ml, équipé d'une ampoule à addition, d'un thermomètre et d'un réfrigérant, on introduit l'acide éthyl malonique (1.00 g; 7.57 mmoles) dans 8 ml de dichlorométhane sec. Le ballon est refroidi à 0 °C par l'intermédiaire d'un bain de glace. A cette température, 1.5 équivalents (0.83 ml; 11.35 mmoles) de chlorure de thionyle dilués dans 5 ml de dichlorométhane sont lentement additionnés. Après addition complète, le mélange réactionnel est agité à 0 °C pendant 10 minutes puis chauffé à reflux pendant 2 jours. Après concentration, on isole le chloroformyl éthanoate d'éthyle (0.99 g; 87%) qui est utilisé sans purification supplémentaire dans l'étape suivante.

### Caractérisation du produit

¹H RMN (CDCl₃, 400 MHz) δ : 1.31 (t, J=7.3 Hz, 3H), 3.87 (s, 2H), 4.26 (q, J=7.3 Hz, 2H); IR (film) : 2989, 1802, 1742, 1577, 1468, 1401, 1372, 1323, 1260, 1164, 1035, 982, 940, 859, 652, 612 cm⁻¹; PM : 150.56.

### Synthèse du N-phénylamido éthanoate d'éthyle

A une solution de N-phényl amine (0.25 g; 0.24 ml; 2.66 mmoles) dans 15 ml de dichlorométhane sec sont additionnés 1.25 équivalents (0.46 ml; 3.32 mmoles) de triéthylamine. La solution obtenue est placée à 0 °C par l'intermédiaire d'un bain de glace. A cette température, 1.25 équivalents (0.43 ml; 3.32 mmoles) de chloroformyl éthanoate d'éthyle sont lentement additionnés au moyen d'une seringue. Une solution orange intense est obtenue qui est agitée à 0 °C pendant une nuit (contrôle de l'évolution de la réaction par TLC) (éluant Pentane/Acétate d'éthyle : 1/9), puis, environ 4 ml d'une solution de HCl 10% sont additionnés. Les deux phases sont séparées et la phase organique est lavée à l'eau. Les phases aqueuses réunies sont extraites au dichlorométhane. Les phases organiques sont lavées avec une solution aqueuse saturée en NaCl, séchées sur MgSO₄ anhydre, filtrées et concentrées. La purification est effectuée par chromatographie sur colonne de silice (éluant Pentane/Acétate d'éthyle : 1/9) pour donner quantitativement le composé de l'invention (MCR2P231) (0.55g; 100%).

### Caractérisation du produit

¹H RMN (CDCl₃, 400 MHz) δ : 1.32 (t, J=7.3 Hz, 3H), 3.47 (s, 2H), 4.26 (q, J=7.3 Hz, 2H), 7.12 (t, J=7.3 Hz, 1H), 7.33 (t, J=7.8 Hz, 2H), 7.55 (d, J=7.8 Hz, 2H); ¹³C RMN (CDCl₃, 175 MHz) δ : 13.67, 42.26, 61.38, 119.98, 124.25, 128.55, 137.37, 163.77, 168.70; IR (film) : 3313, 3143, 2985, 1738, 1668, 1603, 1549, 1500, 1445, 1372, 1342, 1244, 1190, 1156, 1033, 906, 844, 757, 695 cm⁻¹; PM : 207.23; MS m/z 207 (M⁺·).

### Exemple 7 : Synthèse du 2,6-diméthyl-N-phénylamido éthanoate d'éthyle

A une solution de 2,6-diméthyl-N-phényl amine (1.31 ml; 10.63 mmoles) dans 60 ml de dichlorométhane sec est additionnée la triéthylamine (1.85 ml; 13.28 mmoles). Le mélange incolore obtenu est refroidi à 0 °C par l'intermédiaire d'un bain de glace et on y ajoute lentement à la seringue le chloroformyl éthanoate d'éthyle (1.3 ml; 10.15 mmoles). Une solution rouge/orange intense est obtenue qui est maintenue sous agitation à 0 °C pendant 1 nuit (contrôle de l'évolution de la réaction par TLC) (éluant Pentane/Acétate d'éthyle : 1/9), puis, environ 4 ml d'une solution de HCl 10% sont additionnés. Les deux phases sont séparées et la phase organique est lavée à l'eau (3x10 ml). Les phases aqueuses réunies sont extraites au dichlorométhane. La phase organique est lavée avec une solution aqueuse saturée en NaCl puis séchée sur MgSO₄ anhydre. Après filtration et concentration, l'amide ester brute est obtenue sous forme d'un solide orangé. La purification est effectuée par recristallisation dans un mélange Pentane/Acétate d'éthyle pour fournir le composé de l'invention (MCR2P255) (1.67 g; 67%) sous forme d'une poudre couleur écru.

### Caractérisation du produit

¹H RMN (CDCl₃, 400 MHz) δ : 1.35 (t, J=7.3 Hz, 3H), 2.20 (s, 6H), 3.50 (s, 2H), 4.25 (q, J=7.3 Hz, 2H), 7.10 (m, 3H), 8.55 (s élargi, 1H); ¹³C RMN (CDCl₃, 175 MHz) δ : 13.98, 18.29, 41.20, 61.67, 127.23, 128.06, 133.50, 135.10, 163.38, 169.66; IR (pastille KBr) : 3226, 3031, 1731, 1666, 1642, 1595, 1536, 1477, 1411, 1397, 1370, 1351, 1264, 1218, 1204, 1166, 1030, 986, 880, 761, 723, 635 cm⁻¹; m.p. = 103 °C; PM : 235.28; MS m/z 235 (M⁺·).

### Exemple 8 : Synthèse du N-méthyl-N-cyclohexamido éthanoate d'éthyle

A une solution de N-méthyl N-cyclohexylamine (0.41 ml; 3.12 mmoles) dans 20 ml de dichlorométhane sec est additionnée la triéthylamine (0.54 ml; 3.91 mmoles). Le mélange incolore obtenu est refroidi à 0 °C par l'intermédiaire d'un bain de glace et on y ajoute lentement à la seringue le chloroformyl éthanoate d'éthyle (0.5 ml; 3.91 mmoles). Une solution rouge/orange intense est obtenue qui est maintenue sous agitation à 0 °C pendant 4 heures (contrôle de l'évolution de la réaction par TLC) (éluant Pentane/Acétate d'éthyle : 1/9), puis, environ 4 ml d'une solution de HCl 10% sont additionnés. Les deux phases sont séparées et la phase organique est lavée à l'eau (3x10 ml). Les phases aqueuses réunies sont extraites au dichlorométhane. La phase organique est lavée avec une solution aqueuse saturée en NaCl puis séchée sur MgSO₄ anhydre. Après filtration et concentration, l'amide ester brute est obtenue. La purification est effectuée par chromatographie sur colonne de silice (éluant Pentane/Acétate d'éthyle : 1/9) pour fournir le composé de l'invention (MCR2P237) (0.71 g; 100%) sous forme d'un liquide orange.

### Caractérisation du produit obtenu

¹H RMN (CDCl₃, 400 MHz) δ: 1.27-1.67 (m, 14H), 2.84 (s, 3H), 3.44 (s, 1H), 3.47 (s, 1H), 4.21 (q, J=7.3 Hz, 2H), 4.40 (m, contribution forme énol); ¹³C RMN (CDCl₃, 175 MHz) δ : 14.52, 24.99, 29.05, 30.14, 41.44, 52.09, 60.48, 65.10, 165.13, 167.06; IR (film) : 2933, 2858, 1741, 1645, 1477, 1450, 1408, 1370, 1326, 1254, 1164, 1098, 1035, 947, 895, 847, 788, 674 cm⁻¹; PM : 227.30.

### Exemple 9 : Tests in vitro

### Effet des produits au niveau de la respiration mitochondriale (effet au niveau des stades III et IV et au niveau du RCR)

Les composés de l'invention sont caractérisés par l'inhibition de la diminution du contenu en ATP, de l'activation de la phospholipase A2 et de l'adhérence des polymorphonucléaires neutrophiles (PMN) sur les cellules endothéliales de veine ombilicale humaine en culture lorsque celles-ci sont incubées dans des conditions hypoxiques.

L'inhibition par les composés de l'invention de la cascade d'activation des cellules endothéliales induites par l'hypoxie et le maintien du contenu en ATP dans les cellules endothéliales sous hypoxie sont obtenus par les composés de l'invention qui préservent l'activité respiratoire mitochondriale. Ceci est confirmé en mesurant l'activité respiratoire exprimée par le contrôle respiratoire (RCR) de mitochondries de foies de rats traités *per os.* Les mitochondries sont isolées selon la méthode décrite par Nowicki et al. , *J. of Cerebral Blood Flow and Metabolism* **2**, pp. 33-40 (1982). La respiration mitochondriale est mesurée par une électrode à oxygène liée à un enregistreur. Le RCR représente le contrôle respiratoire, et représente le rapport entre la consommation d'oxygène en présence de substrat endogène (glutamate/malate) et la consommation après phosphorylation de l'ADP en ATP. Cette technique a été décrite par Chance & William *(Nature* **175**, pp. 1120-1121 (1955)).

Différents composés de l'invention ont été testés à différentes concentrations sur l'activité respiratoire des mitochondries purifiées et obtenues à partir de foies de rats. Les mitochondries ont été préincubées en présence des composés de manière à mesurer la pente des stades III et IV de la respiration tel que décrit dans le document WO98/51291. Le RCR a ensuite été calculé.

Les résultats ci-dessous montrent que les produits de l'exemple 1 et de l'exemple 2 exercent une action sur le RCR avec un effet maximal à 10⁻⁷ mol/l pour le produit de l'exemple 2. Cette augmentation du RCR est principalement due à un effet de diminution du stade IV de la respiration.

Le composé de l'exemple 1 augmente de manière importante le RCR des mitochondries, et ceci parce que cette molécule diminue fortement le stade IV de la respiration. L'effet est optimal à 10⁻⁶ mol/l et diminue avec la concentration pour être négligeable à 10⁻⁹ mol/l.

Le tableau ci-dessous résume l'effet d'autres composés de l'invention avec les concentrations optimales auxquelles ces effets ont été observés.

**Tableau I**

| **Composé** | **Augmentation du RCR (*)** | **Concentration** |
|---|---|---|
| Malonate de cyclopentèn-2-yle et d'éthyle | 10% | 10⁻⁵ M |
| Malonate de cyclopentèn-2-yle et de méthyle | 15% | 10⁻⁶ M |
| N,N'-diphényl malonamide | 5% | 10⁻⁷ M |
| N-phénylamido éthanoate d'éthyle | 6,6% | 10⁻⁶ M |
| 2,6 diméthyl-N-phénylamido étahnoate d'éthyle | 13% | 10⁻⁶ M |
| N-méthyl-N-cyclohexamido éthanoate d'éthyle | 15% | 10⁻⁷ M |

| | | |
|---|---|---|
| (*) : en % par rapport au contrôle porté à 100% | | |

Les résultats montrent que les composés de l'invention sont capables de protéger de façon reproductible la diminution du contenu en ATP induite par l'hypoxie dans les cellules endothéliales. La protection maximale moyenne de 92,5% est observée à une concentration de 10⁻⁴ mol/l pour le composé de l'exemple 2. Cette protection diminue avec la concentration en ce composé pour être négligeable à 10⁻⁸ mol/l. Les résultats montrent également que le composé de l'exemple 1 est capable de protéger de façon reproductible la diminution du contenu en ATP induite par l'hypoxie dans les cellules endothéliales. La protection maximale moyenne de 92,1% est observée à des concentrations de 10⁻⁴ à 10⁻⁵ mol/l. Cette protection diminue avec la concentration en ce composé pour être négligeable à 10⁻⁸ mol/l.

Cette protection est reproductible dans la mesure où l'on retrouve toujours les mêmes évolutions de courbe pour toutes les expériences, et même s'il existe une variabilité entre expériences au niveau de l'effet de l'hypoxie sur le contenu en ATP. Cette variabilité est inhérente au modèle expérimental. En effet, pour chaque expérience, on utilise une culture différente de cellules isolées d'un cordon ombilical différent, et il existe une variabilité de comportement des cellules d'un cordon à l'autre.

### Exemple 10 : Stabilité dans le plasma humain

Les composés des exemples 1 et 2 ont été soumis à des tests de stabilité afin d'évaluer leur comportement au contact du plasma après une incubation de 3 et 24 heures à une température de 37 °C. Ces tests ont été effectués par GC en suivant l'évolution des chromatogrammes des différents esters par rapport à un standard interne en fonction du temps.

L'injection des produits purs en GC/MF a été effectuée dans les conditions suivantes :
- température d'injection : 250 °C,
- température initiale : 40 °C,
- température finale : 250°C,
- gradient de température : 10 °C/min,
- méthode d'injection : split,
- temps de rétention :
   composé de l'exemple 1 : 13,39
   composé de l'exemple 2 : 11,23

Les différents composés ont été préparés de la façon suivante. 10 µl de composé pur ont été mis en solution dans 100 µl de plasma à une température de 37 °C. La solution obtenue est agitée. Après un temps de 3 heures ou de 24 heures, 5 µl de standard interne (N-benzylamine) et 1 ml d'éther sont additionnés à la solution maintenue à 37 °C. L'échantillon est centrifugé pendant 10 min à une vitesse de 13000 tours/min. 1 µl de la phase surnageante (phase éthérée) est injecté en GC. L'échantillon est ensuite analysé par GC. L'analyse des chromatogrammes permet de mettre en évidence qu'après 3 heures au contact de plasma humain maintenu à 37 °C, le pic correspondant aux composés de l'invention est toujours observé. Les composés de l'invention ne subissent donc pas ou peu de dégradations significatives après 3 heures en présence de plasma. Aucun autre pic pouvant correspondre à des produits de métabolisation n'est observé. Après 24 heures au contact du plasma humain, on observe toujours la présence du pic correspondant au composé de l'exemple 1. Cependant, on ne retrouve plus les pics correspondant au composé de l'exemple 2.

### Exemple 11 : Effet in vitro sur l'adhérence des neutrophiles aux cellules endothéliales incubées sous hypoxie

Des cellules endothéliales ont été incubées sous hypoxie en présence de différentes concentrations des composés de l'invention. Après incubation, les cellules sont co-incubées avec une suspension de neutrophiles humains non stimulés. En conditions normoxiques, l'adhérence de neutrophiles aux cellules endothéliales est faible (de 5 à 10%), mais elle augmente de l'ordre de 3 fois sous hypoxie.

L'effet du composé de l'exemple 1 sur l'adhérence des neutrophiles aux cellules endothéliales incubées sous hypoxie est caractérisé par une inhibition de l'adhérence induite par l'hypoxie, et ce de manière dépendante de la concentration entre 10⁻⁵ et 10⁻⁸ mol/l. Un optimum d'activité devrait être obtenu entre 10⁻⁷ et 10⁻⁵ mol/l. De manière similaire, le composé de l'exemple 2 inhibe l'augmentation de l'adhérence des neutrophiles induites par l'hypoxie entre 10⁻⁶ et 10⁻⁸ mol/l avec un maximum de 71% à 10⁻⁸ mol/l.

### Exemple 12 : Effet in vivo sur le foie après traitement de rats per os pendant 5 jours

Les rats ont été traités *per os* soit avec des solutions de concentration croissante de chacun des composés de l'invention, soit avec des lots contenant du DMSO à la même concentration que celle utilisée pour dissoudre les molécules (rats contrôle) à raison d'une fois par jour pendant 5 jours.

A la suite du traitement, les rats sont sacrifiés. Les mitochondries sont isolées à partir du foie et leur activité respiratoire est mesurée toutes les 15 min pendant les 75 min qui suivent la procédure d'isolation.

La concentration optimale en pré-incubation pour le composé de l'exemple 1 est de 10⁻⁶ mol/l (0,236 µg/l). La dose utilisée pour traiter les rats est donc de 2,36 mg/kg, et des doses 5 fois moindres, 5 fois plus élevées et 20 fois plus élevées ont également été testées.

Pour le composé de l'exemple 2, la concentration optimale en pré-incubation est de 10⁻⁷ mol/l (0,0183 µg/ml). Les doses testées sont donc de 0,18 mg/kg. Etant donné que cette dose était faiblement active, des doses 5 fois plus élevées et 20 fois plus élevées ont été également testées.

Le composé de l'exemple 1 augmente de façon dose-dépendante et significative le RCR des mitochondries hépatiques après un traitement de 5 jours avec une augmentation de 25% à 2,36 mg/kg. A plus forte dose, la molécule devient faiblement toxique et le RCR chute légèrement en deçà des valeurs contrôle. Cette augmentation du RCR est due à une nette diminution du stade IV de la respiration.

Le composé de l'exemple 2 augmente de façon dose-dépendante et significative le RCR des mitochondries avec un maximum de 17% à 3,6 mg/kg. Cette augmentation du RCR est également due à une diminution du stade IV de la respiration.

### Exemple 13 : Effet in vivo sur le coeur après traitement de rats per os pendant un jour

Les rats ont été traités per os avec les concentrations de 2,36 mg/kg de composé de l'exemple 1 ou de 3,6 mg/kg de composé de l'exemple 2, soit avec des lots contenant du DMSO à la même concentration que celle utilisée pour dissoudre les molécules (rats contrôle) à raison d'une fois.

Le lendemain, les rats sont sacrifiés, et les mitochondries sont isolées à partir du coeur. leur activité respiratoire est mesurée toutes les 15 minutes pendant les 75 minutes qui suivent la procédure d'isolation.

Le composé de l'exemple 1 augmente le RCR des mitochondries cardiaques après un traitement d'un jour avec une augmentation de 41% à 2,36 mg/kg. Le composé de l'exemple 2 augmente le RCR des mitochondries après un traitement d'un jour avec une augmentation de 56% à 3,6 mg/kg.

### Exemple 14 : Application in vitro pour le traitement de l'apoptose

Des cellules promyélocytaires sont incubées en présence d'agents induisant l'apoptose comme l'étoposide, un inhibiteur de topoisomérase de type II. Après incubation, différents paramètres signes de l'apoptose sont détectables : activation des caspases, libération par les mitochondries du cytochrome c, fragmentation de l'ADN, etc.

Si les cellules sont incubées en présence d'étoposide et en présence du composé de l'exemple 1, on observe une inhibition de l'induction de l'apoptose. Cette inhibition est dépendante de la dose entre 10⁻⁵ et 10⁻⁸ mol/l. Ainsi, l'activation de la caspase 3, la libération par les mitochondries du cytochrome c et la fragmentation de l'ADN sont inhibés.

## Revendications

1. Composé de formule (I), un sel ou une prodrogue de celui-ci correspondant à la formule I : 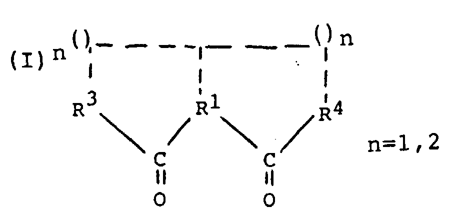 dans laquelle :
- R¹ est CH₂, NH, ou un ligand, de préférence un atome de carbone, formant avec R³ et/ou R⁴ un cycle aromatique ou non aromatique de 5 ou 6 atomes de carbone, comportant éventuellement sur chaque cycle un hétéroatome, de préférence un atome d'azote, d'oxygène ou de soufre;
- R³ et/ou R⁴ représentent :
- une amine de formule 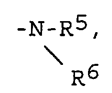 ou
- un groupement de formule -Z-R⁶ dans lesquels :
- Z représente O ou S,
- R⁵ représente H ou un alkyle de 1 à 6 atomes de carbone (de préférence, R⁵ représente un méthyle ou un éthyle),
- R⁶ représente un groupement hydrophobe de préférence terbutyle, allyle ou un cycle aromatique ou non aromatique de 5, 6 ou 7 atomes de carbone comprenant éventuellement un ou plusieurs hétéroatomes et dans lequel les atomes de carbone sont éventuellement substitués par :
- un groupe alkyle R⁷ de 1 à 6 atomes de carbone (de préférence, un méthyle ou un éthyle),
- un groupement cétone,
- un groupement hydroxyle,
- un groupement ester,
- un élément halogène (F, Cl, Br ou I),
- un trifluorométhyle (CF₃), ou
- un groupement de formule 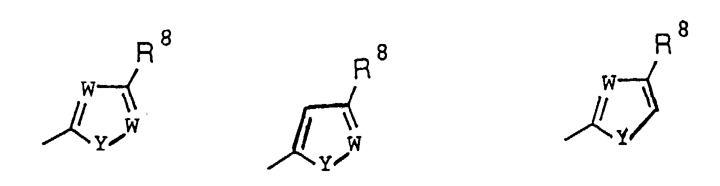
- dans lequel :
- W représente CH ou un atome d'azote,
- Y représente S ou O, et
- R⁸ représente un groupe alkyle de 1 à 6 atomes de carbone, de préférence un méthyle ou un éthyle;
- avec la condition supplémentaire que si R³ forme avec R¹ ledit cycle susmentionné ou que si R³ est soit une amine de formule 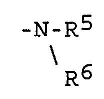 ou le groupement de formule -Z-R⁶ susmentionné, R⁴ représente un alkyle de 1 à 10 atomes de carbone, saturé ou insaturé, comportant éventuellement un ou plusieurs hétéroatomes.

2. Composé selon la revendication 1, caractérisé en ce que R⁵, R⁷ et R⁸ représentent H ou un méthyle, et en ce que R¹ est CH₂.

3. Composé selon la revendication 1 ou 2, caractérisé en ce que R¹ est CH₂ et en ce que R³ et R⁴ représentent une amine de formule 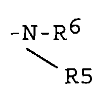 ou un groupement de formule -O-R⁶ dans lesquels R⁶ représente un tertbutyl, un cyclopentène, un cyclohexane ou un benzène dont les atomes de carbone sont éventuellement substitués par un groupe méthyle, un groupe cétone, un groupe hydroxyle, un groupe ester, un élément halogène ou un groupe trifluorométhyle (CF₃).

4. Composé selon l'une quelconque des revendications précédentes, caractérisé en ce qu'il est choisi parmi le groupe constitué par le malonate de dicyclopentèn-2-yle, le 2-propényl 3-oxa-2-oxobicyclo [3.1.0]hexane-1-carboxylate, le malonate de cyclopentèn-2-yle et d'éthyle, le malonate de cyclopentèn-2-yle et de méthyle, la N,N'-diphényl malonamide, le N-phénylamido éthanoate d'éthyle, le 2,6-diméthyl-N-phénylamido éthanoate d'éthyle et le N-méthyl-N-cyclohexylamido éthanoate d'éthyle, éventuellement leurs sels ou des prodrogues de ceux-ci.

5. Composition pharmaceutique comprenant un véhicule pharmaceutique adéquat et un ou plusieurs composé(s) selon l'une quelconque des revendications précédentes.

6. Utilisation du composé selon l'une quelconque des revendications 1 à 4 ou de la composition pharmaceutique selon la revendication 5 pour la préparation d'un médicament destiné au traitement et/ou à la prévention de l'ischémie, des pathologies liées à l'ischémie ou des pathologies liées à des déficiences mitochondriales.

7. Procédé de préparation du composé selon la revendication 1, comprenant les étapes suivantes :
- estérification d'un composé intermédiaire de formule R³-H par un groupement 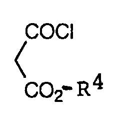 en présence de dicyclohexyl carbodiimide (DCC),
- éventuellement suivie d'une ou plusieurs réactions de cyclisation en présence d'un agent oxydant.
